# EUROPEAN PATENT SPECIFICATION

(11) **EP 2 007 352 B1**
(45) Date of publication and mention of the grant of the patent: **27.04.2011**
(21) Application number: 07759848.0
(22) Date of filing: 30.03.2007
(51) Int. Cl.: A61K 9/00, A61K 31/519, A61P 25/00

(54) **TREATMENT OF PSYCHIATRIC PATIENTS WITH SLEEP DISTURBANCES AND/OR EXCESSIVE DAYTIME SLEEPINESS WITH PALIPERIDONE**
BEHANDLUNG PSYCHIATRISCHER PATIENTEN MIT SCHLAFSTÖRUNGEN UND/ODER ÜBERMÄSSIGER TAGESSCHLÄFRIGKEIT MIT PALIPERIDON
TRAITEMENT À LA PALIPÉRIDONE DE PATIENTS PSYCHIATRIQUES AYANT DES TROUBLES DU SOMMEIL ET/OU UNE SOMNOLENCE EXCESSIVE DANS LA JOURNÉE

(30) Priority: 03.04.2006 US 788764 P; 22.09.2006 US 534632
(43) Date of publication of application: 31.12.2008
(73) Proprietor: Janssen Pharmaceutica NV, 2340 Beerse (BE)
(72) Inventor: EERDEKENS, Marielle-Henriette, B-3650 Dilsen-Stokkem (BE); PALUMBO, Joseph, M., Saint Davids, Pennsylvania 19087-4826 (US); KRAMER, Michelle, San Diego, California 92127 (US)
(74) Representative: Warner, James Alexander
(86) International application number: PCT/US2007/065660
(87) International publication number: WO 2007/118033

(56) References cited:
- WO-A-2004/010981
- LUTHRINGER ET AL: "P.1.b.013 Sleep assessments in patients with schizophrenia following treatment with paliperidone extended-release tablets" EUROPEAN NEUROPSYCHOPHARMACOLOGY, ELSEVIER SCIENCE PUBLISHERS BV, AMSTERDAM, NL, vol. 16, January 2006 (2006-01), page S224, XP005850819 ISSN: 0924-977X

## Description

### FIELD OF THE INVENTION

This invention relates to paliperidone for use in the treatment of psychiatric patients with persistent sleep disturbances and/or excessive daytime sleepiness.

### BACKGROUND OF THE INVENTION

Patients with mental illness often have sleep disturbances including insomnia, excessive sleeping. Sleep disturbances are common patients with schizophrenia, depression and bipolar disorders. For sample patients with schizophrenia appear to have longer sleep latency, a higher number of arousal during sleep and increased periods of wakefulness after sleep onset compared with those in nonpsychiatric control groups. (Tandon et al and Miller et al). Patients being treated for psychotic symptoms may also suffer from excessive daytime sleepiness or drowsiness associated with both sleep disturbance and sedation caused by antipsychotic medications. Additionally, the excessive sedation caused by some antipsychotic medications can result in impaired cognitive functions and other symptoms that interfere with successful treatment of the patient. More importantly sedation can impair a patients quality of life and efforts to lead a normal life.

In WO2004/010981 "[d]osage forms and methods for providing a substantially ascending rate of release of paliperidone are provided. The sustained release dosage forms provide therapeutically effective average steady-state plasma paliperidone concentrations when administered once per day."

We have discovered that, by treating psychiatric patients whosuffer from excessive daytime sleepiness and/or a sleep disturbance with a pharmaceutically effective amount of paliperidone, its pharmaceutically acceptable acid addition salts, enantiomeric forms and esters delivered at a relatively constant plasma level, these the patients will have significantly reduced daytime drowsiness and/or sleep disturbances.

### SUMMARY OF THE INVENTION

In one embodiment the present invention provides paliperidone, its pharmaceutically acceptable acid addition salts, enantiomeric forms and esters thereof in a dosage form that provides relatively low plasma concentration variation of paliperidone, for use in the treatment of a psychiatric patient with excessive daytime sleepiness and/or a sleep disturbance.

This and other objects and advantages of the present invention may be appreciated from a review of the present applications.

### BRIEF DESCRIPTION OF THE FIGURE

Figure 1 provides a graphical presentation of a relatively constant plasma release profile of paliperidone from a controlled release dosage form such as an OROS dosage form as compared to an immediate release form of paliperidone. The data points shown for days 1 show are measured at specific time intervals as is described in Example 4. For days 2 through 6 data points were measured at 2 and 22 hours post administration and constitute and average of the results from each group at each time. The data points for paliperidone shown between days 6 and 7 show multiple time points during the day, illustrating the intraday variance in plasma level concentration of paliperidone.

### DETAILED DESCRIPTION

Sleep disturbances and excessive daytime drowsiness occur in patients suffering from many mental illnesses. These subjects spend an excessive amount of time in a sleep state or unable to maintain a satisfactory degree of wakefulness during a period of the day when wakefulness is required or desired.

As used herein the term "sleep disturbance" shall mean a condition in which an individual has longer sleep latency, a higher number of arousals during sleep, and increased periods of wakefulness after sleep onset (e.g. hypersomnia, dyssomnia and parasomnias). These disorders may be diagnosed using conventional methods such as are commonly employed in a sleep laboratory (e.g. polysomnography).

As used herein the term "Excessive Daytime Sleepiness" (EDS) shall be used interchangeably with the term "pathological somnolence" and shall mean a condition in which an individual feels very drowsy during the day and finds it difficult to resist urge to fall asleep, whether or not the individual has gotten enough nighttime sleep. Excessive daytime sleepiness (EDS) is defined as sleepiness occurring in a situation when an individual would be expected to be awake and alert. Clinically the symptoms of EDS can be quantified and measured in a variety of ways, including but not limited to; the Multiple Sleep Latency Test (MSLT).(See Carskadon MA and Dement WC, Sleep 1982;5 Suppl 2:S67-72), the Maintenance of Wakefulness Test (MWT) (See, Mitler MM, et al. Electroencephalogr Clin Neurophysiol, 1982;53(6):658-61) or the Stanford Sleepiness Scale (SSS) (See, Hoddes E et al., Psychophysiology, 1973;10(4):431-6) (See also, Arand D et al. Sleep, 2005;28(1):123-144). The causes of EDS are multiple and the use of the term EDS herein is not intended to imply any particular cause or etiology. People with EDS frequently doze, nap, or fall asleep in situations where they need or want to be fully awake and alert. The diagnosis can be made when the symptoms of EDS interfere significantly with a person's ability to concentrate and perform daily tasks and routines such as work, family responsibilities, driving a car or operating other hazardous machinery or general quality of life.

The term "psychiatric patient" as used herein, refers to a human, who has been the object of treatment, or experiment for a "mental disorder" and "mental illness" refer to those provided in the Diagnostic and Statistical Manual (DSM III or IV), American Psychological Association (APA). These mental disorders include schizophrenia; bipolar disorder or other disease states in which psychosis, aggressive behavior, anxiety or depression is evidenced. Schizophrenia refers to conditions characterized as schizophrenia, schizoaffective disorder and schizophreniform disorders, in DSM-IV-TR such as category 295.xx. Bipolar Disorder refers to a conditions characterized as a Bipolar Disorder, in DSM-N-TR such as category 296.xx including Bipolar I and Bipolar Disorder II. The DSM-IV-R was prepared by the Task Force on Nomenclature and Statistics of the American Psychiatric Association, and provides clear descriptions of diagnostic categories. Pathologic psychological conditions, which are psychoses or may be associated with psychotic features include, but are not limited to the following disorders that have been characterized in the DSM-IV-TR. Diagnostic and Statistical Manual of Mental Disorders, Revised, 3rd Ed. (1994). The numbers in parenthesis refer to the DSM-IV-TR categories. The skilled artisan will recognize that there are alternative nomenclatures, nosologies, and classification systems for pathologic psychological conditions and that these systems evolve with medical scientific progress.

Examples of pathologic psychological conditions which may be treated include, but are not limited to,. Mild Mental Retardation (317), Moderate Mental Retardation (31R.0), Severe Mental Retardation (318.1), Profound Mental Retardation (318.2), Mental Retardation Severity Unspecified (319), Autistic Disorders (299.00), Rett's Disorder (299.80), Childhood Disintegrative Disorders (299.10), Asperger's Disorder (299.80), Pervasive Developmental Disorder Not Otherwise Specified (299.80), Attention-Deficit/Hyperactivity Disorder Combined Type (314.01), Attention-Deficit/Hyperactivity Disorder Predominately Inattentive Type (314.00), Attention-Deficit/Hyperactivity Disorder Predominately Hyperactive-Impulsive Type (314.01), Attention-Deficit/Hyperactivity Disorder NOS (314.9) Conduct Disorder (Childhood-Onset and Adolescent Type 312.8) Oppositional Defiant Disorder (313.81), Disruptive Behavior Disorder Not Otherwise Specified (312.9), Solitary Aggressive Type (312.00), Conduct Disorder, Undifferentiated Type (312.90), Tourette's Disorder (307.23), Chronic Motor Or Vocal Tic Disorder (307.22), Transient Tic Disorder (307.21), Tic Disorder NOS (307.20), Alcohol Intoxication Delirium (291.0), Alcohol Withdrawal Delirium (291.0), Alcohol-Induced Persisting Dementia (291.2), Alcohol-Induced Psychotic Disorder with Delusions (291.5), Alcohol-Induced Psychotic Disorder with Hallucinations (291.3), Amphetamine or Similarly Acting Sympathomimetic Intoxication (292.89), Amphetamine or Similarly Acting Sympathomimetic Delirium (292.81), Amphetamine or Similarly Acting Sympathomimetic Induced Psychotic with Delusional (292.11), Amphetamine or Similarly Acting Sympathomimetic Induced Psychotic with Hallucinations (292.12), Cannabis-Induced Psychotic Disorder with Delusions (292.11), Cannabis-Induced Psychotic Disorder with Hallucinations (292.12), Cocaine Intoxication (292.89), Cocaine Intoxication Delirium (292.81), Cocaine-Induced Psychotic Disorder with Delusions (292.11), Cocaine-Induced Psychotic Disorder with Hallucinations (292.12), Halluciogen Intoxication (292.89), Hallucinogen Intoxication Delirium (292.81), Hallucinogen-Induced Psychotic disorder with Delusions (292.11), Hallucinogen-Induced Psychotic disorder with Delusions (292.12), Hallucinogen-Induced Mood Disorder (292.84), Hallucinogen-Induced Anxiety Disorder (292.89), Hallucinogen-Related Disorder Not Otherwise Specified (292.9), Inhalant Intoxication (292.89), Inhalant Intoxication Delirium (292.81), Inhalant-Induced Persisting Dementia (292.82), Inhalant-Induced Psychotic Disorder with Delusions (292.11), Inhalant-Induced Psychotic with Hallucinations (292.12), Inhalant-Induced Mood Disorder (292.89), Inhalant-Induced Anxiety Disorder (292.89), Inhalant-Related Disorder Not Otherwise Specified (292.9), Opioid Intoxication Delirium (292.81), Opioid-Induced Psychotic Disorder with Delusions (292.11), Opioid Intoxication Delirium (292.81), Opioid-Induced Psychotic Disorder with Hallucinations (292.12), Opioid-Induced Mood Disorder (292.84), Phencyclidine (PCP) or Similarly Acting Arylcyclohexylamine Intoxication (292.89), Phencyclidine (PCP) or Similarly Acting Arylcyclohexylamine Intoxication Delirium (292.81), Phencyclidine (PCP) or Similarly Acting Arylcyclohexylamine Induced Psychotic Disorder with Delusions (292.11), Phencyclidine (PCP) or Similarly Acting Arylcyclohexylamine Induced Psychotic Disorder with Hallucinations (292.12), Phencyclidine (PCP) or Similarly Acting Arylcyclohexylamine Mood Disorder (292.84), Phencyclidine (PCP) or Similarly Acting Arylcyclohexylamine Induced Anxiety Disorder (292.89), Phencyclidine (PCP) or Similarly Acting Arylcyclohexylamine Related Disorder Not Otherwise Specified (292.9), Sedative, Hypnotic or Anxiolytic Intoxication (292.89), Sedation, Hypnotic or Anxiolytic Intoxication Delirium (292.81), Sedation, Hypnotic or Anxiolytic Withdrawal Delirium (292.81), Sedation, Hypnotic or Anxiolytic Induced Persisting Dementia (292.82), Sedation, Hypnotic or Anxiolytic-Induced Psychotic Disorder with Delusions (292.11), Sedation, Hypnotic or Anxiolytic-Induced Psychotic Disorder with Hallucinations (292.12), Sedation, Hypnotic or Anxiolytic-Induced Mood Disorder (292.84), Sedation, Hypnotic or Anxiolytic-Induced Anxiety Disorder (292.89), Other (or Unknown) Substance Intoxication (292.89), Other (or Unknown) Substance-Induced Delirium (292.81), Other (or Unknown) Substance-Induced Persisting Dementia (292.82), Other (or Unknown) Substance-Induced Psychotic Disorder with Delusions (292.11), Other (or Unknown) Substance-Induced Psychotic Disorder with Hallucinations (292.12), Other (or Unknown) Substance-Induced Mood Disorder (292.84), Other (or Unknown) Substance-Induced Anxiety Disorder (292.89), Other (or Unknown) Substance Disorder Not Otherwise Specified (292.9), Obsessive Compulsive Disorder (300.3), Post-traumatic Stress Disorder (309.81), Generalized Anxiety Disorder (300.02), Anxiety Disorder Not Otherwise Specified (300.00), Body Dysmorphic Disorder (300.7), Hypochondriasis (or Hypochondriacal Neurosis) (300.7), Somatization Disorder (300.81), Undifferentiated Somatoform Disorder (300.81), Somatoform Disorder Not Otherwise Specified (300.81), Intermittent Explosive Disorder (312.34), Kleptomania (312.32), Pathological Gambling (312.31), Pyromania (312.33), Trichotillomania (312.39), and Impulse Control Disorder NOS (312.30), Schizophrenia, Paranoid Type, (295.30), Schizophrenia, Disorganized (295.10), Schizophrenia, Catatonic Type, (295.20), Schizophrenia, Undifferentiated Type (295.90), Schizophrenia, Residual Type (295.60), Schizophreniform Disorder (295.40), Schizoaffective Disorder (295.70), Delusional Disorder (297.1), Brief Psychotic Disorder (298.8), Shared Psychotic Disorder (297.3), Psychotic Disorder Due to a General Medical Condition with Delusions (293.81), Psychotic Disorder Due to a General Medical Condition with Hallucinations (293.82), Psychotic Disorders Not Otherwise Specified (298.9), Major Depression, Single Episode, Severe, without Psychotic Features (296.23), Major Depression, Recurrent, Severe, without Psychotic Features (296.33), Bipolar Disorder, Mixed, Severe, without Psychotic Features (296.63), Bipolar Disorder, Mixed, Severe, with Psychotic Features (296.64), Bipolar Disorder, Manic, Severe, without Psychotic Features (296.43), Bipolar Disorder, Manic, Severe, with Psychotic Features (296.44), Bipolar Disorder, Depressed, Severe, without Psychotic Features (296.53), Bipolar Disorder, Depressed, Severe, with Psychotic Features (296.54), Bipolar II Disorder (296.89), Bipolar Disorder Not Otherwise Specified (296.80), Personality Disorders, Paranoid (301.0), Personality Disorders, Schizoid (301.20), Personality Disorders, Schizotypal (301.22), Personality Disorders, Antisocial (301.7), and Personality Disorders, Borderline (301.83).

The term "a patient in need of treatment" as used herein will refer to any subject or psychiatric patient who currently has or may develop any of the above syndromes or disorders, including any condition or disorder in which the subject spends an excessive amount of time in a sleep state or unable to maintain a satisfactory degree of wakefulness during a period of the day when wakefulness is required or desired or has EDS.

Paliperidone including its pharmaceutically acceptable acid addition salts, enantiomeric forms and esters may be administered for the practice of the present disclosure. Paliperidone is described in US Patent 5,5158,952 incorporated herein by reference. Esters of Paliperidone are describe in US Patent 5,254,556 incorporates herein by reference. Esters of paliperidone include octanoic acid, decanoic acid, dodecanic acid, tetradecanoic acid or hexadecanoic acid (palmitic acid). The currently preferred ester of paliperidone is paliperidone palmitate.

Paliperidone may be formulate with pharmaceutical excipients into a variety of dosage forme as described in US Patent 5,158,952. Paliperidone will in one embodiment of the present invention be provided in an oral dosage forms. Suitable oral dosage forms include but are not limited to controlled release or extended release dosage forms. Currently preferred are once daily extended release dosage forms. Most preferred are extended release OROS oral dosage forms such as those described in US 2004092534, US 20050208132 and US 2005232995 hereby incorporated herein by reference.

Paliperidone according to the present invention should be delivered with a dosage form that will provide low plasma concentration variation (peak to trough) for the patient receiving treatment to reduce excessive daytime sleepiness and/or sleep disturbance. Low plasma concentration variation mean that the intradose variation in plasma concentration (Cmax-Cmin) / Cmax has been minimized. As illustrated in Figure 1 a low plasma concentration variation is achieved by the dosage form being tested (specifically between day 6 and 7). The intradose variation in plasma concentration from the maximum plasma concentration (C max) to the minimum plasma concentration (Cmin) when minimized to reduces excessive daytime sleepiness and/or sleep disturbance. In one embodiment of the invention the reduction in plasma concentration from Cmax to Cmin will be less than about 35 percent, preferably less than about 30 percent, more preferably from about 10 percent to about 30 percent, most preferably From about 15 percent to about 25 percent These ranges are calculated as (Cmax-Cmin) / Cmax from the administration of one dosage form to the next. Preferably the dosing will be once per day.

For the avoidance of doubt "intradose" or "intradose period" refers to the period between the initiation of drug release by a dosage form from its administration until the administration of the next dose of said dosage form in a dosing regiment. Generally, this period will be the period from the administration of one dose to the administration of another dose, except were a dosage form does not release drug immediately upon administration. In this case the period of delay of release of the drug will have to be considered. This adjustment should be considered in establishing Cmin.

Additional Figure 1 illustrates a flat plasma curve. "Flat plasma curve" means a plasma concentration curve that reaches and maintains a substantially constant value after a defined period of time following administration of a dosage form according to the invention. A steady state wherein the absolute values of Cmax and Cmin are relatively constant from day to day is achieved after about 6 days.

"C" means the concentration of drug in blood plasma, or serum, of a subject, generally expressed as mass per unit volume, typically nanograms per milliliter. For convenience, this concentration may be referred to herein as "drug plasma concentration", "plasma drug concentration" or "plasma concentration". The plasma drug concentration at any time following drug administration is referenced as Ctime, as in C9h or C24h, etc. A maximum plasma concentration obtained following administration of a dosage form obtained directly from the experimental data without interpolation is referred to as Cmax. A minimum plasma concentration obtained following administration of a dosage form obtained directly from experimental data without interpolation is referred Cmin..

Persons of skill in the art will appreciate that blood plasma drug concentrations obtained in individual subjects will vary due to inter-patient variability in the many parameters affecting drug absorption, distribution, metabolism and excretion. For this reason, unless otherwise indicated, when a drug plasma concentration is listed, the value listed is the calculated mean value based on values obtained from a groups of subjects tested.

Those of ordinary skill in the art will appreciate that paliperidone, its salts, enatiomers and esters can be administered for all the known uses ofrisperidone. For example paliperidone can be used to treat schizophrenia, schizoaffective disorders, bipolar mania or other disease states in which psychosis, aggressive behavior, anxiety or depression is evidenced (e.g. psychosis associated with dementia, post traumatic stress syndrome, etc.).

As used herein the term "subject", refers to an animal, preferably a mammal, and most preferably a human, who has been the object of treatment, observation or experiment.

The term **"therapeutically effective amount"** as used herein, means that amount of active compound or pharmaceutical agent that elicits the biological or medicinal response in human that is being sought by a researcher, medical doctor or other clinician, which includes alleviation of the symptoms of the disease or disorder being treated.

Those of skill in the treatment of diseases could easily determine the effective amount of paliperidone to administer for the treatment of the diseases listed above. In general it is contemplated that an effective amount would be from about 0.01mg/kg to about 2 mg/kg body weight. In one embodiment of present disclosure wherein paliperidone is orally administered a dosage form to a subject once a day is preferred. The mg of compound delivered in such a dosage form to the patient may be from 0.25 to about 20 mg (e.g. 0.25 mg, 0.5 mg, 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, and 20 mg) per oral dosage form.

### EXAMPLE 1

### Paliperidone Capsule Shaped Tablet, Trilayer 2 mg System

A dosage form adapted, designed and shaped as an osmotic drug delivery device was manufactured as follows: 120 g of paliperidone, 7325 g of polyethylene oxide with average molecular weight of 200,000, and 2000 g of sodium chloride, USP were added to a fluid bed granulator bowl. Next a binder solution was prepared by dissolving 400 g of hydroxypropylmethyl cellulose identified as 2910 having an average viscosity of 5 cps in 7,600 g of water. The dry materials were fluid bed granulated by spraying with 4,000 g of binder solution. Next, the wet granulation was dried in the granulator to an acceptable moisture content, and sized using by passing through a 7-mesh screen. Next, the granulation was transferred to a blender and mixed with 5 g of butylated hydroxytoluene as an antioxidant and lubricated with 50 g of stearic acid.

Next, a second drug compartment composition was prepared as follows: 280 g of paliperidone and 9165 g of polyethylene oxide with average molecular weight of 200,000 were added to a fluid bed granulator bowl. Next a binder solution was prepared by dissolving 400 g of hydroxypropylmethyl cellulose identified as 2910 having an average viscosity of 5 cps in 7,600 g of water. The dry materials were fluid bed granulated by spraying with 4,000 g of binder solution. Next, the wet granulation was dried in the granulator to an acceptable moisture content, and sized using by passing through a 7-mesh screen. Next, the granulation was transferred to a blender and mixed with 5 g of butylated hydroxytoluene as an antioxidant and lubricated with 50 g of stearic acid.

Next, a push composition was prepared as follows: first, a binder solution was prepared. 15.6 kg of polyvinylpyrrolidone identified as K29-32 having an average molecular weight of 40,000 was dissolved in 104.4 kg of water. Then, 24 kg of sodium chloride and 1.2 kg of ferric oxide were sized using a Quadro Comil with a 21-mesh screen. Then, the screened materials and 88.44 kg of Polyethylene oxide (approximately 7,000,000 molecular weight) were added to a fluid bed granulator bowl. The dry materials were fluidized and mixed while 46.2 kg of binder solution was sprayed from 3 nozzles onto the powder. The granulation was dried in the fluid-bed chamber to an acceptable moisture level. The coated granules were sized using a Fluid Air mill with a 7-mesh screen. The granulation was transferred to a tote tumbler, mixed with 15 g of butylated hydroxytoluene and lubricated with 294 g magnesium stearate.

Next, the paliperidone drug compositions for the first and the second compartments and the push composition were compressed into trilayer tablets. First, 50 mg of the paliperidone compartment one composition was added to the die cavity and pre-compressed, then 50 mg of the paliperidone compartment two composition was added to the die cavity and pre-compressed, then 100 mg of the push composition was added and the layers were pressed into a 3/16" diameter longitudinal, deep concave, trilayer arrangement.

The trilayered arrangements were coated with a subcoat laminate. The wall forming composition comprised 70% hydroxypropyl cellulose identified as EF, having an average molecular weight of 80,000 and 30% of polyvinylpyrrolidone identified as K29-32 having an average molecular weight of 40,000. The wall-forming composition was dissolved in anhydrous ethyl alcohol, to make an 8% solids solution. The wall-forming composition was sprayed onto and around the bilayered arrangements in a pan coater until approximately 20 mg of laminate was applied to each tablet.

The trilayered arrangements were coated with a semi-permeable wall. The wall forming composition comprised 99% cellulose acetate having a 39.8% acetyl content and 1% polyethylene glycol comprising a 3.350 viscosity-average molecular weight. The wall-forming composition was dissolved in an acetone:water (95:5 wt:wt) co solvent to make a 5% solids solution. The wall-forming composition was sprayed onto and around the bilayered arrangements in a pan coater until approximately 40 mg of membrane was applied to each tablet.

Next, two 25 mil (0.6 mm) exit passageways were laser drilled through the semi-permeable wall to connect the drug layer with the exterior of the dosage system. The residual solvent was removed by drying for 144 hours as 45 Deg C and 45% humidity. After drilling, the osmotic systems were dried for 4 hours at 45 Deg C to remove excess moisture.

The dosage form produced by this manufacture was designed to deliver 2 mg of paliperidone in an ascending delivery pattern from two drug-containing cores. The first core contained 1.2% paliperidone, 73.25% polyethylene oxide possessing a 200,000 molecular weight, 20% sodium chloride, USP, 5% hydroxypropylmethyl cellulose having an average viscosity of 5 cps, 0.05% butylated hydroxytoluene, and 0.5% stearic acid. The second drug core contained 2.8% paliperidone, 91.65% polyethylene oxide possessing a 200,000 molecular weight, 5% hydroxypropylmethyl cellulose having an average viscosity of 5 cps, 0.05% butylated hydroxytoluene, and 0.5% stearic acid. The push composition comprised 73.7% polyethylene oxide comprising a 7,000,000 molecular weight, 20% sodium chloride, 5% polyvinylpyrrolidone possessing an average molecular weight of 40,000, 1% ferric oxide, 0.05% butylated hydroxytoluene, and 0.25% magnesium stearate. The semi permeable wall was comprised of 99% cellulose acetate of 39.8% acetyl content and 1% polyethylene glycol. The dosage form comprised two passageways, 25 mils (0.6 mm) on the center of the drug side.

### EXAMPLE 2

### Paliperidone Capsule Shaped Tablet, Trilayer 3 mg System

A dosage form adapted, designed and shaped as an osmotic drug delivery device is manufactured as follows: 2.246 kg of paliperidone, 87.2 kg of polyethylene oxide with average molecular weight of 200,000 and 24 kg of sodium chloride, USP are added to a fluid bed granulator bowl. Paliperidone amount includes a 4% manufacturing excess to compensate for losses during processing. Next, a binder solution is prepared by dissolving 7.2 kg of polyvinylpyrrolidone identified as K29-32 having an average molecular weight of 40,000 in 52.8 kg of water. The dry materials are fluid bed granulated by spraying with 50 kg of binder solution. Next, the wet granulation is dried in the granulator to the acceptable moisture content. Then, the dried granulation is sized using a Fluid Air mill with a 7-mesh screen. The granulation is transferred to a tote tumbler, mixed with 60 g of butylated hydroxytoluene and lubricated with 600 g stearic acid.

Next, a second drug compartment composition is prepared as follows: 5.242 kg of paliperidone, 0.06 kg of yellow ferric oxide and 108.2 kg of polyethylene oxide with average molecular weight of 200,000 are added to a fluid bed granulator bowl. Paliperidone amount includes a 4% manufacturing excess to compensate for losses during processing. Next, a binder solution is prepared by dissolving 7.2 kg of polyvinylpyrrolidone identified as K29-32 having an average molecular weight of 40,000 in 52.8 kg of water. The dry materials are fluid bed granulated by spraying with 50 kg of binder solution. Next, the wet granulation is dried in the granulator to the acceptable moisture content. Then, the dried granulation is sized using a Fluid Air mill with a 7-mesh screen. The granulation is transferred to a tote tumbler, mixed with 60 g of butylated hydroxytoluene and lubricated with 600 g stearic acid.

Next, a push composition is prepared as follows: first, a binder solution is prepared. 30 kg of polyvinylpyrrolidone identified as K29-32 having an average molecular weight of 40,000 is dissolved in 200.8 kg of water. Then, 100 kg of sodium chloride and 5 kg of red ferric oxide are sized using a Quadro Comil with a 21-mesh screen. Then, the screened materials and 368.50 kg of Polyethylene oxide (approximately 7,000,000 molecular weight) are added to a fluid bed granulator bowl. The dry materials are fluidized and mixed while 192 kg of binder solution is sprayed from 3 nozzles onto the powder. The granulation is dried in the fluid-bed chamber to an acceptable moisture level. The coated granules are sized using a Fluid Air mill with a 7-mesh screen. The granulation is transferred to a tote tumbler, mixed with 240 g of butylated hydroxytoluene and lubricated with 1200 g stearic acid.

Next, the paliperidone drug compositions for the first and the second compartments and the push composition are compressed into trilayer tablets. First, 50 mg of the paliperidone compartment one composition is added to the die cavity and pre-compressed, then 50 mg of the paliperidone compartment two composition is added to the die cavity and pre-compressed, then 100 mg of the push composition is added and the layers are pressed into a 3/16" diameter longitudinal, deep concave, trilayer arrangement.

The trilayered arrangements are coated with a subcoat laminate. The wall forming composition comprises 95% hydroxyethylcellulose and 5% of polyethylene glycol comprising a 3.350 viscosity-average molecular weight. The wall-forming composition is dissolved in water, to make an 8% solids solution. The wall-forming composition is sprayed onto and around the bilayered arrangements in a pan coater until approximately 10 mg of laminate is applied to each tablet.

The trilayered arrangements are coated with a semi-permeable wall. The wall forming composition comprises 99% cellulose acetate having a 39.8% acetyl content and 1% polyethylene glycol comprising a 3.350 viscosity-average molecular weight.

The wall-forming composition is dissolved in an acetone:water (95:5 wt:wt) co solvent to make a 5% solids solution. The wall-forming composition is sprayed onto and around the bilayered arrangements in a pan coater until approximately 45 mg of membrane is applied to each tablet.

Next, two 25 mil (0.64 mm) exit passageways are laser drilled through the semi-permeable wall to connect the drug layer with the exterior of the dosage system. The residual solvent is removed by drying for 144 hours as 45 C. and 45% humidity followed with approximate 1 hour at 45 C. to remove excess moisture.

The dosage form produced by this manufacture is designed to deliver 3 mg of paliperidone in an ascending delivery pattern from two drug-containing cores.

### EXAMPLE 3

### Paliperidone Capsule Shaped Tablet, Trilayer 9 mg System

A dosage form adapted, designed and shaped as an osmotic drug delivery device is manufactured as follows: 6.64 kg of paliperidone, 82.86 kg of polyethylene oxide with average molecular weight of 200,000 and 24 kg of sodium chloride, USP are added to a fluid bed granulator bowl. Paliperidone amount includes a 2.5% manufacturing excess to compensate for losses during processing. Next, a binder solution is prepared by dissolving 7.2 kg of polyvinylpyrrolidone identified as K29-32 having an average molecular weight of 40,000 in 52.8 kg of water. The dry materials are fluid bed granulated by spraying with 50 kg of binder solution. Next, the wet granulation is dried in the granulator to the acceptable moisture content. Then, the dried granulation is sized using a Fluid Air mill with a 7-mesh screen. The granulation is transferred to a tote tumbler, mixed with 60 g of butylated hydroxytoluene and lubricated with 600 g stearic acid.

Next, a second drug compartment composition is prepared as follows: 15.50 kg of paliperidone, 0.018 kg of black iron oxide, and 98.20 kg of polyethylene oxide with average molecular weight of 200,000 are added to a fluid bed granulator bowl. Paliperidone amount includes a 2.5% manufacturing excess to compensate for losses during processing. Next, a binder solution is prepared by dissolving 7.2 kg of polyvinylpyrrolidone identified as K29-32 having an average molecular weight of 40,000 in 52.8 kg of water. The dry materials are fluid bed granulated by spraying with 50 kg of binder solution. Next, the wet granulation is dried in the granulator to the acceptable moisture content. Then, the dried granulation is sized using a Fluid Air mill with a 7-mesh screen. The granulation is transferred to a tote tumbler, mixed with 60 g ofbutylated hydroxytoluene and lubricated with 600 g stearic acid.

Next, a push composition is prepared as follows: first, a binder solution is prepared. 30 kg of polyvinylpyrrolidone identified as K29-32 having an average molecular weight of 40,000 is dissolved in 200.8 kg of water. Then, 100 kg of sodium chloride and 5 kg of red ferric oxide are sized using a Quadro Comil with a 21-mesh screen. Then, the screened materials and 368.50 kg of Polyethylene oxide (approximately 7,000,000 molecular weight) are added to a fluid bed granulator bowl. The dry materials are fluidized and mixed while 192 kg of binder solution is sprayed from 3 nozzles onto the powder. The granulation is dried in the fluid-bed chamber to an acceptable moisture level. The coated granules are sized using a Fluid Air mill with a 7-mesh screen. The granulation is transferred to a tote tumbler, mixed with 240 g of butylated hydroxytoluene and lubricated with 1200 g stearic acid.

Next, the paliperidone drug compositions for the first and the second compartments and the push composition are compressed into trilayer tablets. First, 50 mg of the paliperidone compartment one composition is added to the die cavity and pre-compressed, then 50 mg of the paliperidone compartment two composition is added to the die cavity and pre-compressed, then 100 mg of the push composition is added and the layers are pressed into a 3/16" diameter longitudinal, deep concave, trilayer arrangement.

The trilayered arrangements are coated with a subcoat laminate. The wall forming composition comprises 95% hydroxyethylcellulose and 5% of polyethylene glycol comprising a 3.350 viscosity-average molecular weight. The wall-forming composition is dissolved in water, to make an 8% solids solution. The wall-forming composition is sprayed onto and around the bilayered arrangements in a pan coater until approximately 10 mg of laminate is applied to each tablet.

The trilayered arrangements are coated with a semi-permeable wall. The wall forming composition comprises 99% cellulose acetate having a 39.8% acetyl content and 1% polyethylene glycol comprising a 3.350 viscosity-average molecular weight. The wall-forming composition is dissolved in an acetone:water (95:5 wt:wt) co solvent to make a 5% solids solution. The wall-forming composition is sprayed onto and around the bilayered arrangements in a pan coater until approximately 45 mg of membrane is applied to each tablet.

Next, two 25 mil (0.64 mm) exit passageways are laser drilled through the semi-permeable wall to connect the drug layer with the exterior of the dosage system. The residual solvent is removed by drying for 144 hours as 45 C. and 45% humidity followed with approximate 1 hour at 45 C. to remove excess moisture. The dosage form produced by this manufacture is designed to deliver 9 mg of paliperidone in an ascending delivery pattern from two drug-containing cores.

### EXAMPLE 4

### Determination of Plasma Profile of Paliperidone OROS formulation

### Dosage and Administration

RISPERIDAL® (isperidone IR) brand risperidone was administered to subjects (in these example the subjects are human patients). The initial dose of risperidone was 2 mg/day and titrated to 4mg/day on Day 2. Because the bioavailability of ER OROS paliperidoen is approximately 30% that of an oral solution of paliperidone 12mg ER OROS paliperidone dose was selected to administered as an equivalent dose with respect to plasma levels to 4mg IR risperidone. The ER OROS paliperidone was provided as 2mg tablets made substantially as described in Example 1.

Subjects in each group were given a once-daily oral dose, consisting of six (6) 2mg capsules, every morning during the washout and double-blind treatment periods. Study medication was administered sitting in an upright position with 200mL of water immediately after a standardized breakfast eaten between approximately 8:00 a.m. and 10:00 a.m.

### Study Evaluations

Subjects were required to complete a washout period prior to participation in the study. Subjects who successfully completed the washout period and continued to meet eligibility criteria were randomly assigned in equal number to one of three treatment groups (the results of two the treatment groups are provided in this example); (1) Paliperidone OROS or (2) risperidone immediate release. During the double-blind treatment phase (days 1 to 6), study mediation was administered after a standard breakfast. All meals during the study, including the washout and treatment periods, were standardized, consisting of a normal sodium diet with approximately 2,500 Kcal per 24 hours. The subject's total fluid intake was monitored and was not to exceed 3,000 mL per 24 hours. Subjects were to have no more than 3 methylxanthin-containing beverages(e.g. cola, tea coffee) daily. Soft drinks were permitted as long as they did not contain quinine or caffeine and did not increase the permitted total daily calories allowance. Other foods including chocolate, grapefruit, grapefruit juice Seville oranges and quinine containing beverages were not allowed. Strenuous physical activity was not permitted during the study.

### Pharmacokinetic Evaluations

At time points during the double-blind treatment period until the end of the study on Day 7, blood samples were obtained for measurement of risperidone, paliperidone and active moiety concentrations in plasma. In each treatment group, a venous blood sample fo 5 mL was drawn to obtain about 2 to 3mL of plasma. The collection schedule was as follows:
On Day 1, immediately before dosing and at 1, 2, 3, 4, 6, 8, 12, 18, and 22 hours postdose:
On Day 2 through Day 5, immediately before dosing and at 2 and 22 hours postdose;
On Day 6, immediately before dosing and at 1, 2, 3, 4, 6, 8, 12, 18, 22, and 24 hours postdose (on Day 7).

Plasma concentrations of risperidone and paliperidone were determined by using a validated liquid chromatography coupled to mass spectrometry/mass spectrometry method

For the subjects who received IR risperidone, the active moiety concentrations were calculated as the sum of the risperidone and paliperidone concentrations.

### EXAMPLE 5

### Sleep Study

**Purpose:** Nighttime sleep disturbance and daytime drowsiness are common in patients with schizophrenia, and can be a significant concern for patients that is not always effectively addressed with pharmacologic treatment. Paliperidone extended-release tablet (paliperidone ER) is an investigational psychotropic/antipsychotic agent, with once-daily dosing, that has been shown to be efficacious and well-tolerated in 6-week trials of patients with schizophrenia¹⁻³. Effects of paliperidone ER on changes in sleep architecture (Dataset 1) were studied in patients with schizophrenia-related insomnia, and the clinical importance of these findings was further assessed by patient-rated changes in quality of sleep and daytime drowsiness (Dataset 2) in patients with schizophrenia.

**Methods**: Data were collected in multicenter, double-blind, randomized and placebo-controlled studies in patients with either stable (Dataset 1) or acute (Dataset 2) schizophrenia. In Dataset 1 (n=42) paliperidone ER 9mg or placebo were administered daily for 2 weeks. Polysomnography (PSG) variables were assessed at baseline and endpoint. Other sleep measures included Leeds Sleep Evaluation Questionnaire (LSEQ). Dataset 2 (n=1326) was a post-hoc analysis of pooled data from three, 6-week studies¹⁻³ with paliperidone ER 3mg, 6mg, 9mg, 12mg, 15mg, or placebo daily. The paliperidone ER capsules were prepared as described in Example 2 and 3 above. Patients dosed with 6mg were provided with two 3mg tablets. Patients dosed with 12g were provided with one 3mg and one 9mg tablet. Patients dosed with 15mg were provided a 9mg and two 3mg tablets. Subjects completed Visual Analogue Scale (VAS) assessments of sleep quality ('How well have you slept in the past 7 nights?') and daytime drowsiness ('How often have you felt drowsy in the past 7 days?').

**Results:** Dataset 1 analysis set (n=36) consisted of 67% male, mean±SD age=32.2y±7.3, baseline mean±SD PANSS total score=62.9±11.2, mean PSG measured latency to persistent sleep of 58.7±45.8 minutes, sleep onset latency of 49.9±41.1 minutes, and a severity of insomnia confirmed by a baseline mean Sleep Efficiency Index (SEI) of 76±13.4%. Treatment with paliperidone ER resulted in clinically robust and statistically significant improvements in sleep, including decreases in latency to persistent sleep (41.9±23.7 minutes) and sleep onset latency (35.9±20.6 minutes). SEI was improved (77.5±15.6%), total sleep time increased (370.4±74.3 minutes), Stage II sleep duration increased (219.3±52.5 minutes), and REM sleep duration increased (80.1±24.6 minutes). Paliperidone ER was without significant effects on slow wave sleep. No statistically significant difference was observed for any change in LSEQ score. The clinical relevance of these findings was supported by Dataset 2 (ITT set: n=1306, 62% male, mean±SD age=38.1y±10.9, baseline mean±SD PANSS total score=93.5±11.8). All 5 doses of paliperidone ER provided significantly greater improvements in quality of sleep compared with placebo, as rated by VAS assessment (mean±SD baseline/change at endpoint: paliperidone ER 3mg=56.4±28.8/9.0±34.5; paliperidone ER 6mg=53.5±30.3/11.1±33.7; paliperidone ER 9mg=56.5±28.7/11.4±33.0; paliperidone ER 12mg=55.7±28.0/9.7±33.7; paliperidone ER 15mg=53.2±29.1/11.3±33.2; placebo=52.4±29.3/0.6±35.9 [p<0.05]). No significant daytime drowsiness was associated with paliperidone ER treatment (mean±SD baseline/change at endpoint: paliperidone ER 3mg=29.4±26.6/-2.9±28.1; paliperidone ER 6mg=28.3±25.1/-0.9±27.9; paliperidone ER 9mg=31.5±27.3/-4.1±32.4; paliperidone ER 12mg=32.5±26.8/-2.9±31.0; paliperidone ER 15mg=38.5±30.3/- 3.8±34.5; placebo=32.3±27.3/-3.0±30.0 [p<0.05]).

**Conclusions:** These studies suggest that paliperidone ER treatment resulted in improvements in sleep architecture and sleep continuity and patient-rated sleep quality without producing or exacerbating the daytime drowsiness that is typically often associated with therapies effective for the treatment of insomnia.

### References

1. Mardcr S, et al. Neuropsychopharmacol 2005; 30: S200
2. Kane J, et al. Neuropsychopharmacol 2005; 30: S191-2
3. Davidson M, et al. Schizophr Res 2006; 81: S43

## Claims

1. Paliperidone, its pharmaceutically acceptable acid addition salts, enantiomeric forms and esters thereof in a dosage form that provides a relatively low plasma concentration variation of paliperidone, for use in the treatment of a psychiatric patient with excessive daytime sleepiness and/or a sleep disturbance.

2. Paliperidone, its pharmaceutically acceptable acid addition salts, enantiomeric forms and esters thereof for use according to claim 1 wherein the psychiatric patient has excessive daytime sleepiness.

3. Paliperidone, its pharmaceutically acceptable acid addition salts, enantiomeric forms and esters thereof for use according to claim 1 wherein the psychiatric patient has a sleep disturbance.

4. Paliperidone, its pharmaceutically acceptable acid addition salts, enantiomeric forms and esters thereof for use according to claim 1 wherein the psychiatric patient has a mental disorder or mental illness selected from the group consisting of schizophrenia, bipolar diseases and other disease states in which psychosis aggressive behavior anxiety or depression is evidenced.

5. Paliperidone, its pharmaceutically acceptable acid addition salts, enantiomeric forms and esters thereof for use according to claim 4 wherein the psychiatric patient has a schizophrenia type mental disorder or mental illness selected from the group consisting of schizophrenia, schizoaffective disorder and schizophreniform disorders.

6. Paliperidone, its pharmaceutically acceptable acid addition salts, enantiomeric forms and esters thereof for use according to claim 4 wherein the psychiatric patient has a bipolar mental disorder or mental illness selected from the group consisting of Bipolar I disorder and Bipolar II disorder.

7. Paliperidone, its pharmaceutically acceptable acid addition salts, enantiomeric forms and esters thereof for use according to claim 1 wherein the low plasma concentration variation comprises a change in plasma concentration (Cmax-Cmin/Cmax) of less than about 30 percent.

8. Paliperidone, its pharmaceutically acceptable acid addition salts, enantiomeric forms and esters thereof for use according to claim 1 wherein the low plasma concentration variation comprises a change in plasma concentration (Cmax-Cmin/Cmax) of from about 10 percent to about 30 percent.

9. Paliperidone, its pharmaceutically acceptable acid addition salts, enantiomeric forms and esters thereof for use according to claim 1 wherein the low plasma concentration variation comprises a change in plasma concentration (Cmax-Cmin/Cmax) of from about 15 percent to about 25 percent.

## Patentansprüche

1. Paliperidon, seine pharmazeutisch annehmbaren Säureadditionssalze, entantiomere Formen und Ester davon in einer Dosierform, die für eine relativ geringe Schwankung der Plasmakonzentration von Paliperidon sorgt, zur Verwendung bei der Behandlung eines psychiatrischen Patienten mit übermäßiger Schläfrigkeit während des Tages und/oder mit Schlafstörung.

2. Paliperidon, seine pharmazeutisch annehmbaren Säureadditionssalze, entantiomere Formen und Ester davon zur Verwendung nach Anspruch 1, worin der psychiatrische Patient an übermäßiger Schläfrigkeit während des Tages leidet.

3. Paliperidon, seine pharmazeutisch annehmbaren Säureadditionssalze, entantiomere Formen und Ester davon zur Verwendung nach Anspruch 1, worin der psychiatrische Patient eine Schlafstörung hat.

4. Paliperidon, seine pharmazeutisch annehmbaren Säureadditionssalze, entantiomere Formen und Ester davon zur Verwendung nach Anspruch 1, worin der psychiatrische Patient an einer Geistesstörung oder Geisteskrankheit aus der Schizophrenie, bipolare Störungen und andere Krankheitszustände, die mit Psychosen, aggressivem Verhalten, Angst oder Depression einhergehen, umfassenden Gruppe leidet.

5. Paliperidon, seine pharmazeutisch annehmbaren Säureadditionssalze, entantiomere Formen und Ester davon zur Verwendung nach Anspruch 4, worin der psychiatrische Patient an einer Geistesstörung vom Schizophrenie-Typ oder einer Geisteskrankheit aus der Schizophrenie, schizoaffektive Störung und schizophreniforme Störungen umfassenden Gruppe leidet.

6. Paliperidon, seine pharmazeutisch annehmbaren Säureadditionssalze, entantiomere Formen und Ester davon zur Verwendung nach Anspruch 4, worin der psychiatrische Patient an einer bipolaren Geistesstörung oder einer Geisteskrankheit aus der bipolare Störung I und bipolare Störung II umfassenden Gruppe leidet.

7. Paliperidon, seine pharmazeutisch annehmbaren Säureadditionssalze, entantiomere Formen und Ester davon zur Verwendung nach Anspruch 1, worin die geringe Schwankung der Plasmakonzentration eine Veränderung der Plasmakonzentration (Cmax-Cmin/Cmax) von weniger als ca. 30 Prozent umfasst.

8. Paliperidon, seine pharmazeutisch annehmbaren Säureadditionssalze, entantiomere Formen und Ester davon zur Verwendung nach Anspruch 1, worin die geringe Schwankung der Plasmakonzentration eine Veränderung der Plasmakonzentration (Cmax-Cmin/Cmax) von ca. 10 Prozent bis ca. 30 Prozent umfasst.

9. Paliperidon, seine pharmazeutisch annehmbaren Säureadditionssalze, entantiomere Formen und Ester davon zur Verwendung nach Anspruch 1, worin die geringe Schwankung der Plasmakonzentration eine Veränderung der Plasmakonzentration (Cmax-Cmin/Cmax) von ca. 15 Prozent bis ca. 25 Prozent umfasst.

## Revendications

1. Palipéridone, ses sels d'addition d'acide pharmaceutiquement acceptables, formes énantiomères et esters de celle-ci sous une forme pharmaceutique qui fournit une variation de concentration plasmatique de palipéridone relativement faible, destinés à être utilisés dans le traitement d'un patient psychiatrique atteint de somnolence de jour excessive et/ou d'un trouble du sommeil.

2. Palipéridone, ses sels d'addition d'acide pharmaceutiquement acceptables, formes énantiomères et esters de celle-ci destinés à être utilisés selon la revendication 1, le patient psychiatrique étant atteint de somnolence de jour excessive.

3. Palipéridone, ses sels d'addition d'acide pharmaceutiquement acceptables, formes énantiomères et esters de celle-ci destinés à être utilisés selon la revendication 1, le patient psychiatrique étant atteint d'un trouble du sommeil.

4. Palipéridone, ses sels d'addition d'acide pharmaceutiquement acceptables, formes énantiomères et esters de celle-ci destinés à être utilisés selon la revendication 1, le patient psychiatrique étant atteint d'un trouble mental ou d'une maladie mentale choisis dans le groupe constitué par la schizophrénie, les maladies bipolaires ou les autres états pathologiques dans lesquels se manifeste une psychose, un comportement agressif, de l'anxiété ou une dépression.

5. Palipéridone, ses sels d'addition d'acide pharmaceutiquement acceptables, formes énantiomères et esters de celle-ci destinés à être utilisés selon la revendication 4, le patient psychiatrique étant atteint d'un trouble mental de type schizophrénie ou d'une maladie mentale choisie dans le groupe constitué par la schizophrénie, un trouble shizo-affectif et les troubles schizophréniformes.

6. Palipéridone, ses sels d'addition d'acide pharmaceutiquement acceptables, formes énantiomères et esters de celle-ci destinés à être utilisés selon la revendication 4, le patient psychiatrique étant atteint d'un trouble mental bipolaire ou d'une maladie mentale choisie dans le groupe constitué par le trouble bipolaire I et le trouble bipolaire II.

7. Palipéridone, ses sels d'addition d'acide pharmaceutiquement acceptables, formes énantiomères et esters de celle-ci destinés à être utilisés selon la revendication 1, la faible variation de concentration plasmatique comprenant un changement de concentration plasmatique [(Cₘₐₓ-Cₘᵢₙ)/Cₘₐₓ] inférieur à environ 30 pour cent.

8. Palipéridone, ses sels d'addition d'acide pharmaceutiquement acceptables, formes énantiomères et esters de celle-ci destinés à être utilisés selon la revendication 1, la faible variation de concentration plasmatique comprenant un changement de concentration plasmatique [(Cₘₐₓ-Cₘᵢₙ)/Cₘₐₓ] d'environ 10 pour cent à environ 30 pour cent.

9. Palipéridone, ses sels d'addition d'acide pharmaceutiquement acceptables, formes énantiomères et esters de celle-ci destinés à être utilisés selon la revendication 1, la faible variation de concentration plasmatique comprenant un changement de concentration plasmatique [(Cₘₐₓ-Cₘᵢₙ)/Cₘₐₓ] d'environ 15 pour cent à environ 25 pour cent.
